# EUROPÄISCHE PATENTANMELDUNG

(11) **EP 0 926 491 A1**
(43) Veröffentlichungstag der Anmeldung: **30.06.1999**
(21) Anmeldenummer: 97811021.1
(22) Anmeldetag: 24.12.1997
(51) Int. Cl.: G01N 29/24, B06B 1/06

(54) **Ultraschallmessvorrichtung,-system und Verwendung derselben**

(71) Anmelder: SULZER INNOTEC AG, CH-8401 Winterthur (CH)
(72) Erfinder: Moser, Urs Dr, CH-8006 Zürich (CH)
(74) Vertreter: Heinen, Detlef

(57) **Zusammenfassung**

Eine Ultraschallmessvorrichtung für ein bildgebendes Ultraschallsystem hat einen Trägerkörper (2), der um eine Drehachse (A) dreh- oder schwenkbar ist, und eine Mehrzahl von auf dem Trägerkörper (2) angeordneten Wandlerelementen (3) zum Aussenden und Empfangen von Ultraschallsignalen. Mindestens zwei der Wandlerelemente (3) sind bezüglich der Umfangsrichtung des Trägerkörpers (2) und bezüglich der durch die Drehachse (A) festgelegten axialen Richtung relativ zueinander versetzt angeordnet.

## Beschreibung

Die Erfindung betrifft eine Ultraschallmessvorrichtung, ein Ultraschallsystem sowie die Verwendung einer solchen Vorrichtung bzw. eines solchen Systems gemäss dem Oberbegriff des unabhängigen Patentanspruchs der jeweiligen Kategorie.

Bei den modernen minimalinvasiven Untersuchungs- und Behandlungsmethoden kommt der Bildgebung mittels Ultraschall eine wesentliche Bedeutung zu, unter anderem weil sie ohne ionisierende Strahlung, wie z. B. Röntgenstrahlung, auskommt und somit für den Patienten und den Arzt unbelastend ist.

Eine der zahlreichen Anwendungen der Bildgebung mittels Ultraschall ist beispielsweise die transösophagale Herzabbildung, die hauptsächlich zu Diagnosezwecken eingesetzt wird. Viele der heute üblichen Ultraschallsysteme erzeugen jedoch nur zweidimensionale Bilder und es ist oft selbst für Spezialisten sehr schwierig, anhand solcher zweidimensionaler Bilder die dreidimensionale Anatomie zu analysieren. Deshalb werden grosse Anstrengungen unternommen, die Anatomie mittels dreidimensionaler Ultraschallbilder darzustellen.

Vom Stand der Technik sind auch schon solche Ultraschallsysteme bekannt, die dreidimensionale Bilder erzeugen (im folgenden auch als 3D-Ultraschallsysteme bezeichnet). Bei einem bekannten 3D-Ultraschallsystem wird eine Sonde mit einem Ultraschallwandler verwendet, der bezüglich zweier Achsen schwenkbar ist. Durch Schwenken des Wandlers um diese beiden Achsen, was üblicherweise elektronisch gesteuert erfolgt, wird die abzubildende Anatomie, z. B. eine Herzkammer, zunächst bereichsweise und sequentiell mittels Ultraschall abgetastet und anschliessend aus den derart erhaltenen Echosignalen in einer Datenverarbeitungsanlage ein dreidimensionales Bild rekonstruiert.

Ein wesentlicher Nachteil dieser bekannten 3D-Ultraschallsysteme besteht darin, dass die benötigten Abtastzeiten für eine ausreichende räumliche Auflösung des Bildes relativ lang sind. Bekannte 3D-Systeme brauchen typischerweise einige, beispielsweise mindestens fünf, Sekunden für die Erstellung eines einzigen dreidimensionalen Bildes. Für Anwendungen wie beispielsweise die Navigation oder Lokalisierung von innerhalb des Körpers benötigten Instrumenten, z. B. Herzkathetern zur Ablation von Reizleitungen im Herzen, sind derart lange Zeiten für die Bilderstellung unbefriedigend, weil sie dem Bedürfnis einer schnellen und genauen Lokalisierung der momentanen Lage der Instrumente im Körper entgegenstehen.

Ausgehend von diesem Stand der Technik ist es daher eine Aufgabe der Erfindung, eine Ultraschallmessvorrichtung vorzuschlagen, die eine deutlich schnellere Erstellung eines räumlich gut aufgelösten Bildes ermöglicht. Insbesondere soll es die Vorrichtung auch ermöglichen, Ultraschalldaten zu liefern, aus denen dreidimensionale Bilder des abzubildenden Volumens rekonstruiert werden können. Ferner ist es eine Aufgabe der Erfindung ein entsprechendes Ultraschallsystem bereitzustellen, das insbesondere für die rasche Erstellung dreidimensionaler Bilder geeignet ist.

Die diese Aufgaben lösende Ultraschallmessvorrichtung bzw. das diese Aufgaben lösende Ultraschallsystem ist durch die Merkmale des unabhängigen Anspruchs der jeweiligen Kategorie gekennzeichnet. Eine bevorzugte Verwendung der Vorrichtung bzw. des Systems ergibt sich aus dem unabhängigen Verwendungsanspruch.

Erfindungsgemäss wird also eine Ultraschallmessvorrichtung für ein bildgebendes Ultraschallsystem vorgeschlagen, welche Vorrichtung einen Trägerkörper umfasst, der um eine Drehachse dreh- oder schwenkbar ist, sowie eine Mehrzahl von auf dem Trägerkörper angeordneten Wandlerelementen zum Aussenden und Empfangen von Ultraschallsignalen. Mindestens zwei der Wandlerelemente sind bezüglich der Umfangsrichtung des Trägerkörpers und bezüglich der durch die Drehachse festgelegten axialen Richtung relativ zueinander versetzt angeordnet. Durch diese Anordnung der Wandlerelemente ist es möglich, mehrere Wandlerelemente parallel oder im wesentlichen parallel zu betreiben, sodass mehrere im wesentlichen parallele Schichten des abzubildenden Volumens gleichzeitig oder fast gleichzeitig abtastbar sind.

Diese Anordnung der Wandlerelemente bringt folglich einen enormen Zeitgewinn hinsichtlich der für die Bilderstellung notwendigen Datenerfassung mit sich. Daraus resultiert eine signigikante Verkürzung der für die Bilderstellung benötigten Zeit. Hierdurch wird es möglich, dreidimensionale Bilder wesentlich schneller, z. B. in deutlich weniger als einer Sekunde, zu erstellen.

Mit der erfindungsgemässen Ultraschallvorrichtung sind in Verbindung mit modernen Datenverarbeitungsanlagen Bildfrequenzen dreidimensionaler Ultraschallbilder von mindestens 10-20 Bildern pro Sekunde erreichbar. Somit können 3D-Bilder - zumindest näherungsweise - in Echtzeit erstellt werden. Dies ist insbesondere für solche Anwendungen von bildgebenden Ultraschallsystemen ein grosser Vorteil, bei denen anhand der Ultraschallbilder die Lokalisierung oder Navigation von Instrumenten im Körper erfolgt, weil eine nahezu kontinuierliche Visualisierung der Korperstrukturen und Instrumente möglich ist.

In einer bevorzugten Ausgestaltung sind die Wandlerelemente in mindestens zwei bezüglich der Umfangsrichtung des Trägerkörpers versetzten Gruppen angeordnet, wobei die Wandlerelemente innerhalb jeder Gruppe bezüglich der axialen Richtung benachbart angeordnet sind, also nicht alle auf der gleichen Höhe in axialer Richtung liegen.

Dadurch, dass die mindestens zwei Gruppen von Wandlerelementen bezüglich der Umfangsrichtung des Trägerkörpers versetzt angeordnet sind, kann im Betriebszustand durch Schwenken oder Rotieren des Trägerkörpers zunächst ein paralleles Abtasten durch die Wandlerelemente der erste Gruppe erfolgen und anschliessend ein Abtasten durch die Wandlerelemente der zweiten Gruppe. Durch diese bauliche Anordnung lässt sich insbesondere auch eine bessere räumliche Auflösung erzielen wie weiter hinten näher erläutert wird.

Das erfindungsgemässe Ultraschallsystem umfasst eine erfindungsgemässe Ultraschallmessvorrichtung und ermöglicht somit die rasche Erstellung von Ultraschallbildern, insbesondere von dreidimensionalen Bildern. Es ermöglicht somit die dreidimensionale, zumindest näherungsweise kontinuierliche Darstellung von Instrumenten und Körperstrukturen unter Beibehaltung der bekannten Vorteile der Bildgebung mittels Ultraschall.

Eine bevorzugte Verwendung des erfindungsgemässen Ultraschallsystems bzw. der erfindungsgemässen Ultraschallmessvorrichtung ist die Darstellung, insbesondere die dreidimensionale Darstellung, von Teilen des menschlichen oder tierischen Körpers, insbesondere des Herzens oder Teilen davon.

Weitere vorteilhafte Massnahmen und bevorzugte Ausgestaltungen der Erfindungsgegenstände ergeben sich aus den abhängigen Ansprüchen.

Im folgenden wird die Erfindung anhand der Zeichnung und anhand von Ausführungsbeispielen näher erläutert. In der schematischen, nicht massstäblichen Zeichnung zeigen:
- Fig. 1:: eine schematische Darstellung eines Ausführungsbeispiels der erfindungsgemässen Ultraschallmessvorrichtung,
- Fig. 2:: eine Querschnittsdarstellung des Ausführungsbeispiels entlang der Schnittlinie II-II in Fig. 1,
- Fig. 3:: eine aufgeklappte Darstellung des Ausführungsbeispiels aus Fig. 1 bzw. Fig. 2, und
- Fig. 4:: eine Blockdarstellung eines Ausführungsbeispiels des erfindungsgemässen Ultraschallsystems.

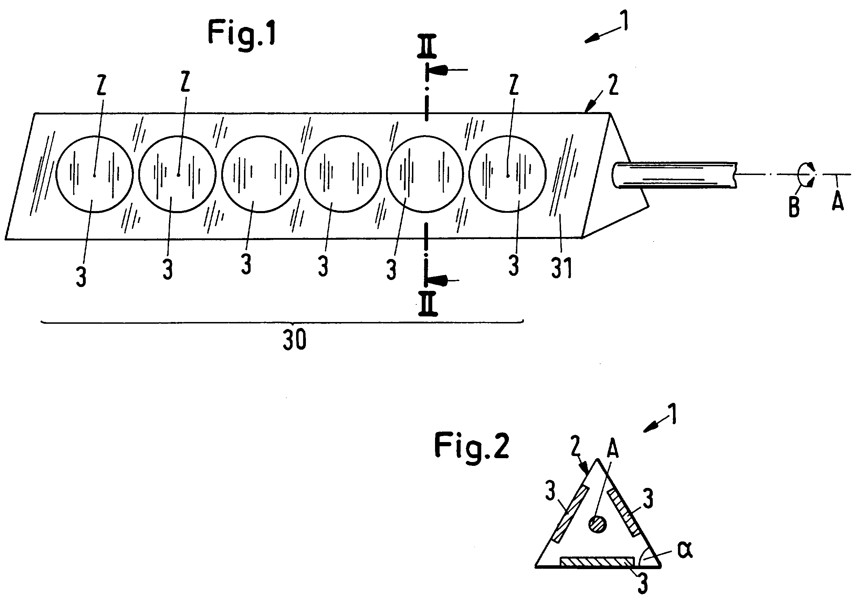

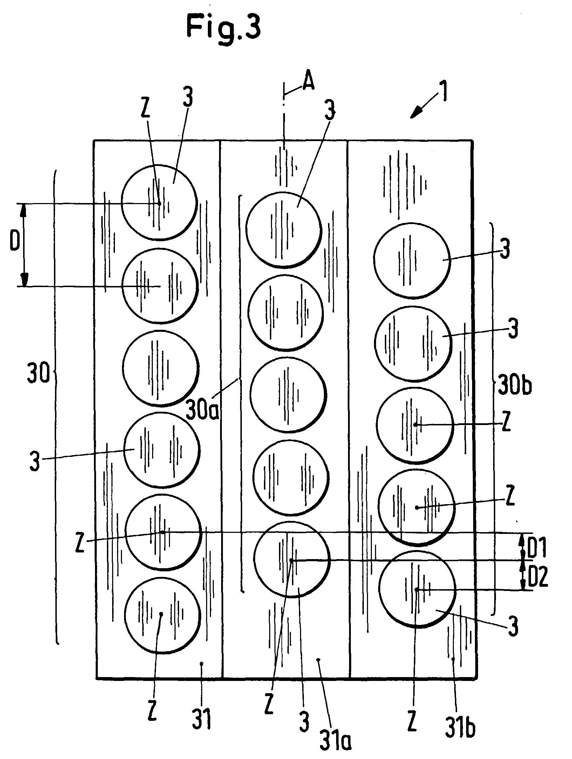

Unter Bezugnahme auf die Fig. 1-3 wird zunächst ein bevorzugtes Ausführungsbeispiel der erfindungsgemässen Ultraschallvorrichtung, die gesamthaft mit dem Bezugszeichen 1 versehen ist, näher erläutert. Fig. 1 zeigt in schematischer Darstellung eine Ansicht und Fig. 2 einen Querschnitt entlang der Schnittlinie II-II in Fig. 1 dieses Ausführungsbeispiels. Fig. 3 zeigt dieses Ausführungsbeispiel noch in einer aufgeklappten Darstellung. Es versteht sich, dass diese aufgeklappte Darstellung nur dem besseren Verständnis dient.

Die Ultraschallmessvorrichtung 1 umfasst einen Trägerkörper 2, der um eine Drehachse A dreh- oder schwenkbar ist wie dies der Doppelpfeil B in Fig. 1 andeutet. Auf dem Trägerkörper 2 sind mehrere Wandlerelemente 3 angeordnet, die jeweils Ultraschallsignale aussenden und empfangen können. Üblicherweise sendet jedes Wandlerelement 3 ein Ultraschallsignal in Form eines Impulses aus und empfängt das von der abzubildenden Körperstruktur reflektierte Echosignal. Bei diesem Ausführungsbeispiel sind die Wandlerelemente 3 in drei Gruppen 30,30a,30b (siehe Fig. 3) - angeordnet, wobei die Gruppen 30,30a,30b bezüglich der Umfangsrichtung des Trägerkörpers 2 versetzt sind. Üblicherweise ist der Trägerkörper 2 in einer Schutzhülle angeordnet oder von einer Ummantelung umgeben.

Um beispielsweise bei transösophagale Anwendungen ein leichteres Einführen der Ultraschallmessvorrichtung 1 zu ermöglichen, kann der Trägerkörper 2 flexibel, also biegsam ausgestaltet sein und/oder an die Anatomie angepasst sein.

Bei dem hier beschriebenen Ausführungsbeispiel weist der Trägerkörper 2 drei Messflächen 31,31a,31b auf, die jeweils planar ausgestaltet sind und jeweils eine der Gruppen 30 bzw. 30a bzw. 30b von Wandlerelementen 3 enthalten. Dabei sind die jeweils benachbarten Messflächen 31,31a,31b gewinkelt zueinander angeordnet, das heisst, jeweils benachbarte Messflächen 30,30a,30b stossen unter einem Winkel α aneinander. Bei dem hier beschriebenen Ausführungsbeispiel sind die drei Messflächen 31,31a,31b relativ zueinander so angeordnet, dass der Trägerkörper 2 senkrecht zur Drehachse A eine dreieckige Querschnittsfläche aufweist wie dies in Fig. 2 dargestellt ist.

Innerhalb jeder Gruppe 30 bzw. 30a bzw. 30b sind die zu dieser Gruppe 30,30a,30b gehörenden Wandlerelemente 3 bezüglich der axialen Richtung benachbart angeordnet. Die axiale Richtung ist durch die Drehachse A festgelegt, das heisst, mit-der axialen Richtung ist die Richtung der Drehachse A gemeint. Aus konstruktiven Gründen sind die Wandlerelemente 3 innerhalb jeder Gruppe 30,30a,30b vorzugsweise linear angeordnet. Die Zentren Z der Wandlerelemente 3, die zu der gleichen Gruppe 30 oder 30a oder 30b gehören, liegen dabei auf einer Geraden, die parallel zur Drehachse A verläuft.

Bei dem bevorzugten Ausführungsbeispiel gemäss den Fig. 1-3 sind die Wandlerelemente 3 derart angeordnet, dass die Zentren Z der Wandlerelemente 3 der einen Gruppe, z. B 30, bezüglich den Zentren Z der Wandlerelemente 3 der oder einer anderen Gruppe 30a oder 30b in axialer Richtung versetzt sind. Diese Anordnung ist am besten aus Fig. 3 ersichtlich. Die Zentren Z zweier unmittelbar benachbarter Wandlerelemente 3 der gleichen Gruppe z. B der Gruppe 30 (im folgenden als erste Gruppe 30 bezeichnet) haben zueinander einen axialen Abstand D. Die Wandlerelemente 3 der in Umfangsrichtung nächsten Gruppe 30a (im folgenden als zweite Gruppe 30a bezeichnet) sind so angeordnet, dass ihre Zentren Z bezüglich der Zentren Z der Wandlerelemente 3 der ersten Gruppe 30 jeweils in axialer Richtung um einen Betrag D1 versetzt sind. Die Wandlerelemente 3 der im folgenden als dritte Gruppe bezeichneten Gruppe 30b sind so angeordnet, dass ihre Zentren Z bezüglich den Zentren Z der Wandlerelemente 3 der zweiten Gruppe 30a jeweils um einen Betrag D2 in axialer Richtung versetzt sind. Die Funktion dieser Anordnung wird weiter hinten im Zusammenhang mit der Beschreibung des Betriebszustands der Ultraschallmessvorrichtung 1 erläutert.

Besonders bevorzugt ist der axiale Versatz D1 bzw. D2 zwischen den Zentren Z der entsprechenden Wandlerelemente 3 benachbarter Gruppen 30,30a,30b im wesentlichen gleich dem Quotienten aus dem axialen Abstand D der Zentren Z zweier benachbarter Wandlerelemente 3 der gleichen Gruppe 30 oder 30a oder 30b und der Anzahl der Messflächen 31,31a,31b bzw. der Anzahl der Gruppen 30,30a,30b. Bei dem hier erläuterten Ausführungsbeispiel ist also vorzugsweise D1 = D2 = D/3, das heisst die Zentren Z der Wandlerelemente der zweiten Gruppe 30a sind jeweils bezüglich den Zentren Z der Wandlerelemente 3 der ersten Gruppe 30 um ein Drittel des Abstands D in axialer Richtung versetzt. Analog sind die Zentren Z der Wandlerelemente 3 der dritten Gruppe 30b bezüglich den entsprechenden Zentren Z der Wandlerelemente 3 der zweiten Gruppe 30a jeweils um ein Drittel des Abstands D in axialer Richtung versetzt.

Als Wandlerelemente 3 eignen sich alle ansich bekannten oder handelsüblichen Ultraschallwandler (Transducer), beispielsweise mit einstückigen piezoelektrischen Kristallen. Auch sind solche Wandlerelemente 3 geeignet, die mehrere Sende/Empfangsflächen aufweisen, beispielsweise in Form konzentrischer Ringe. Die Wandlerelemente (3) können alle gleich ausgestaltet sein, oder es können auch unterschiedliche Wandlerelemente (3) verwendet werden.

Im Betriebszustand, das heisst zur Aufnahme von Ultraschallbildern, wird der Trägerkörper 2 der Ultraschallmessvorrichtung 1 um seine Drehachse A vorzugsweise rotiert. Je nach konkreter Ausgestalltung der Vorrichtung ist es prinzipiell aber auch möglich, anstelle der Rotation eine Schwenkbewegung um die Drehachse A durchzuführen.

Bei der Rotation passieren die drei Gruppen 30,30a,30b von Wandlerelementen 3 sukzessive die abzubildende Körperstruktur. Dabei senden die Wandlerelemente 3, von einer Steuereinrichtung gesteuert, Ultraschallimpulse aus und empfangen in der Folge das von der Körperstruktur reflektierte Echosignal. Durch die spezielle Anordnung der Wandlerelemente 3 in Gruppen 30,30a,30b können dabei verschiedenen im wesentlichen parallele Ebenen der abzubildenden Struktur gleichzeitg mit Ultraschall abgetastet werden. Dazu werden jeweils die Wandlerelemente 3, die zu der gleichen Gruppe 30,30a,30b gehören, zeitgleich und parallel betrieben. Die Wandlerelemente 3, die zu der gleichen Gruppe 30,30a,30b gehören, erzeugen also eine Mehrzahl von Ultraschallimpulsen, die sich im wesentlichen parallel zueinander ausbreiten. Dadurch werden durch eine Gruppe von Wandlerelemente 3 gleichzeitig soviele verschiedene parallele Ebenen der abzubildenden Struktur abgetastet wie die entsprechende Gruppe 30,30a,30b Wandlerelemente 3 aufweist. Durch dieses parallel Abtasten mehrerer Ebenen lässt sich die für die Datenakquisition benötigte Zeit ganz erheblich verkürzen, sodass eine dreidimensionale Bildgebung zumindest näherungsweise in Echtzeit erfolgen kann.

Je nach der speziellen Anordnung der Wandlerelemente 3 bezüglich der axialen Richtung ist es auch möglich, die zu einer Gruppe 30, 30a, 30b gehörenden Wandlerelemente 3 nicht exakt parallel im Sinne von zeitgleich zu betreiben, sondern im wesentlichen parallel, womit gemeint ist, dass zwischen dem Aktivieren (Aussenden des Ultraschallimpulses) zweier Wandlerelemente 3 eine Zeitspanne liegen kann, die jedoch deutlicher kleiner ist als die Laufzeit, die ein Ultraschallsignal benötigt, um von dem Wandlerelement 3 zur abzubildenden Struktur und von dort als Echosignal wieder zurück zum gleichen Wandlerelement 3 zu gelangen. Das heisst, zwei Wandlerelemente werden als "im wesentlichen parallel betrieben" bezeichnet, wenn das zweite Wandlerelement zum Aussenden eines Ultraschallimpulses aktiviert wird, bevor das erste Wandlerelement das von ihm ausgesandte Signal als Echosignal empfängt. Auch bei diesem "im wesentlichen parallelen" Betrieb resultiert im Vergleich zur sukzessiven Abtastung ein deutlicher Zeitgewinn für die Erfassung der Ultraschalldaten. Der im wesentlichen parallele Betrieb ist insbesondere vorteilhaft, wenn beispielsweise die Wandlerelemente 3 einer Gruppe, abweichend von der Darstellung in Fig. 3, nicht alle bezüglich der Umfangsrichtung des Trägerkörpers 2 die gleiche Position haben, sondern auch innerhalb einer Gruppe zueinander in Umfangsrichtung versetzt sind.

Der axiale Versatz der verschiedenen Gruppen 30,30a,30b relativ zueinander (siehe Fig. 3) ist insbesondere im Hinblick auf die erzielbare räumliche Auflösung besonders vorteilhaft, wie im folgenden näher erläutert wird. Beim Betrieb des in den Fig. 1-3 dargestellten Ausführungsbeispiels sei ohne Beschränkung der Allgemeinheit angenommen, dass zunächst die erste Gruppe 30 an der abzubildenden Struktur vorbei gedreht wird. Dabei tasten die sechs zugehörigen Wandlerelemente 3 sechs verschiedenen, im wesentlichen parallele Ebenen der abzubildenden Struktur ab. In der Folge passiert bei weiterer Rotation des Trägerkörpers 2 die zweite Gruppe 30a die abzubildende Struktur und tastet dabei mit ihren fünf Wandlerelementen 3 fünf weitere im wesentlichen parallele Ebenen der Körperstruktur ab, wobei diese Ebenen aufgrund der axialen Versetzung der Wandlerelemente 3 zwischen den von der ersten Gruppe 30 abgetasteten Ebenen liegen. Schliesslich passiert die dritte Gruppe 30b die abzubildende Struktur und tastet dabei mit ihren fünf Wandlerelementen 3 ebenfalls fünf parallele Ebenen der Struktur ab, wobei diese Ebenen zwischen den von der zweiten und der ersten Gruppe 30 bzw. 30a abgetasteten Ebenen liegt. Somit wird die abzubildende Struktur während einer Umdrehung des Trägerkörpers 2 insgesamt in sechzehn verschiedenen, im wesentlichen parallelen Ebenen abgetastet. Die derart gesammelten Daten werden in einer Datenverarbeitungseinheit in ein Bildformat, vorzugsweise in ein dreidimensionales Bildformat, umgesetzt. Dabei werden z. B. die Informationen bezüglich der ersten Dimension der abzubildenden Struktur aus der durch die mechanische Rotation des Trägerkörpers 2 bedingten Abtastung einer Ebene gewonnen, die Information bezüglich der zweiten Dimension aus der von den unterschiedlichen Wandlerelementen 3 kommenden Signale und die Information bezüglich der dritten Dimension aus den Laufzeiten der Echosignale. Zur Bildrekonstruktion können aus der Bildgebung ansich bekannte Datenverarbeitungs- und Bildrekonstruktionsprozeduren eingesetzt werden.

Der axiale Versatz der einzelnen Gruppen 30,30a,30b relativ zueinander ist zwar aufgrund der Erhöhung des räumlichen Auflösevermögens besonders vorteilhaft, es sind aber auch Ausgestaltungen der erfindungsgemässen Ultraschallmessvorrichtung ohne diesen axialen Versatz möglich. Dabei können beispielsweise die verschiedenen Gruppen jeweils die gleichen Ebenen der abzubildenden Struktur abtasten, sodass die verschiedenen Ebenen pro Umdrehung des Trägerkörpers mehrfach abgetastet werden. Auf diese Weise lässt sich die Redundanz der erfassten Ultraschalldaten erhöhen, oder es ist möglich, während einer Umdrehung des Trägerkörpers 2 Daten für mehrere Bilder zu sammeln.

Bevorzugt wird pro Umdrehung des Trägerkörpers 2 ein Ultraschallbild erstellt, sodass die Rotationsfrequenz des Trägerkörpers 2 geich der Bildfrequenz ist. In Kombination mit modernen, schnellen Datenverarbeitungseinheiten können beispielsweise pro Sekunde 10-20 dreidimensionale Ultraschallbilder erzeugt werden, d. h. der Trägerkörper 2 rotiert mit einer Frequenz von 10-20 Umdrehungen pro Sekunde. Dies ermöglicht eine näherungsweise kontinuierliche dreidimensionale Darstellung der abzubildenden Körperstruktur, was insbesondere im Hinblick auf die Lokalisierung und Navigation von Instrumenten im Körper von grossem Vorteil ist.

Es versteht sich, dass zahlreiche Variationen bezüglich der konkreten Ausgestaltung der erfindungsgemässen Ultraschallmessvorrichtung 1 möglich sind, von denen hier nur einige in nicht abschliessender Aufzählung genannt seien.

Beispielsweise kann der Trägerkörper 2 nur zwei oder aber mehr als drei Gruppen 30,30a,30b von Wandlerelementen 3 aufweisen. So kann der Trägerkörper in sinngemäss gleicher Weise wie vorne beschrieben mehr als drei Messflächen 31,31a,31b aufweisen, von denen jede jeweils eine Gruppe von Wandlerelementen 2 aufweist. Weiterin können die einzelnen Messflächen 31,31a,31b auch gekrümmt ausgestaltet sein. Auch ist es möglich, den Trägerkörper 2 im wesentlichen zylindrisch auszugestalten und die einzelnen Wandlerelemente bzw. Gruppen von Wandlerelementen über die Mantelfläche des Zylinders zu verteilen. Ferner können anstelle der in den Fig. 1 und 3 gezeigten Anordnung der Wandlerelemente 3 einer Gruppe 30,30a,30b auf einer Geraden parallel zur Drehachse A auch andere Anordnungen der Wandlerelemente 3 innerhalb einer Gruppe realisiert werden. So können die Wandlerelemente 3 einer Gruppe bzw. ihre Zentren Z beispielsweise auf einer gekrümmten Linie liegen oder auf einer Geraden, die nicht parallel zur Drehachse A liegt oder auf einer Zickzacklinie.

Insbesondere wenn der Trägerkörper 2 zylindrisch ausgestaltet ist, können die einezelnen Wandlerelemente 3 derart auf der Mantelfläche des Trägerkörpers 2 angeordnet sein, dass ihre Zentren Z auf einer Schraubenlinie liegen. Für diese Anordnung eignet sich insbesondere der vorne erläuterte im wesentlichen parallele Betrieb. Auch können die einzelnen Wandlerelemente ohne besondere geometrische Anordnung über die Mantelfläche des Trägerkörpers 2 verteilt angeordnet sein.

Die Anzahl der Wandlerelemente 3 bzw. der Gruppen von Wandlerelementen 3 und die Anzahl der Wandlerelemente 3 in einer Gruppe richtet sich je nach Anwendung nach der Ausdehnung der abzubildenden Struktur in Richtung der Drehachse A und nach dem gewünschten räumlichen Auflösungsvermögen. Dabei kann das Auflösungsvermögen wie vorne beschrieben erhöht werden, indem die verschiedenen Wandlerelemente 3 bzw. Gruppen von Wandlerelementen 3 relativ zueinander in axialer Richtung versetzt angeordnet werden. Dies bringt den Vorteil mit sich, dass das Auflösungsvermögen in Richtung der Drehachse nicht durch die Anzahl beziehungsweise den axialen Abstand der Wandlerelemente 3 innerhalb einer Gruppe begrenzt ist, sondern durch die Gesamtzahl der Wandlerelemente 3, die unterschiedliche Ebenen abtasten. Deshalb können die einzelnen Wandlerelemente 3 relativ gross, beispielsweise mit einem Durchmesser von bis zu einem Centimeter (effektive Apertur) ausgestaltet werden, ohne das Zugeständnisse an das räumliche Auflösungsvermögen vonnöten sind. Solche relativ grossen Wandlerelemente 3 sind insbesondere aus schallphysikalischen Gründen vorteilhaft.

Die Übertragung der Signale zwischen den im Betriebszustand rotierenden Wandlerelementen 3 und dem üblicherweise ortsfesten Ultraschallsystem kann beispielsweise über Schleifkontakte wie Schleifringe oder andere geeignete Kommunikationsmittel, z. B. kontaktlose Koppler wie optoelektronische, elektromagnetische oder induktive Koppler, erfolgen.

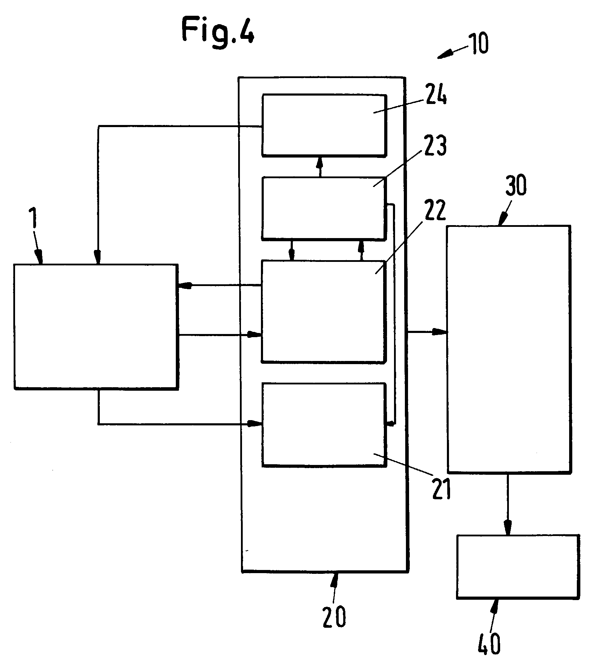

In Fig. 4 sind in einer schematischen Blockdarstellung wesentliche Teile eines Ausführungsbeispiels eines erfindungsgemässen Ultraschallsystems zur Bildgebung, insbesondere zur Erstellung dreidimensionaler Bilder dargestellt. Das Ultraschallsystem ist gesamthaft mit dem Bezugszeichen 10 bezeichnet. Es umfasst eine erfindungsgemässe Ultraschallmessvorrichtung 1, eine Steuer- und Versorgungseinrichtung 20 für die Ultraschallmessvorrichtung 1 und eine Datenverarbeitungseinheit 30, welche die von der Ultraschallmessvorrichtung 1 gelieferten Signale erfasst und derart verarbeitet, dass sie als Bild darstellbar sind. Die dreidimensionale Darstellung kann beispielsweise mittels dafür geeigneter Visualisierungsmittel 40 wie einem Monitor, einem Bildschirm gegebenenfalls in Verbindung mit einer 3D-Brille oder einem sogenannten Head Mounted Display (HMD) erfolgen. Der Signal- bzw. Datenfluss ist in Fig. 4 symbolisch durch die Pfeile angedeutet. Die Steuer- und Versorgungseinrichtung 20 umfasst ein Steuermodul 22, welches mit der Ultraschallmessvorrichtung 1 in Verbindung steht und nicht dargestellte Antriebsmittel zur Rotation des Trägerkörpers 2 steuert. Diese Antriebsmittel können Teil der Ultraschallmessvorrichtung 1 sein oder auch ausserhalb dieser vorgesehen sein. Das Steuermodul 22 kann ferner so ausgestaltet sein, dass es nicht dargestellte Positionierungsmittel zum Erfassen der Position der Ultraschallmessvorrichtung versorgt und steuert. Ferner umfasst die Steuer- und Versorgungseinrichtung 20 ein Zeitgebermodul 23, welches mit dem Steuermodul 22 kommunizieren kann und zusammen mit diesem über eine Sendereinheit 24 die einzelnen Wandlerelemente 3 so ansteuert, dass einerseits während der Rotation des Trägerkörpers 2 die entsprechenden Wandlerelemente 3 jeweils zu den richtigen Zeitpunkten zum Aussenden von Ultraschallimpulsen aktiviert werden und dass andererseits jeweils mehrere Wandlerelemente 3, insbesondere die zu der gleichen Gruppe gehörenden Wandlerelemente 3, parallel oder im wesentlichen parallel betrieben werden. Die von den Wandlerelementen 3 nach der Reflexion an der abzubildenden Struktur empfangenen Ultraschallechosignale werden einem Empfangsmodul 21 zugeführt, wo eine Vorverarbeitung der erfassten Daten, z. B. Verstärkung und/oder Filterung, erfolgt. Die Daten werden dann der Datenverarbeitungseinheit 30 zugeführt, die diese Daten digitalisiert und mittels ansich bekannter Prozeduren zur zwei- bzw. dreidimensionalen Bildgebung in ein Format umwandelt, das eine dreidimensionale Visualisierung ermöglicht. Das Empfangsmodul 21 sowie die Datenverarbeitungseinheit 30 stehen ebenfalls mit dem Zeitgebermodul 23 in Verbindung, um eine korrekte räumliche und zeitliche Zuordnung der Daten zu ermöglichen.

Ferner ist eine in Fig. 4 nicht dargestellte Benutzerschnittstelle zur Bedienung des Ultraschallsystems 10 vorgesehen.

Die erfindungsgemässe Ultraschallmessvorrichtung 1 bzw. das erfindungsgemässe Ultraschallsystem 10 sind insbesondere zur Bildgebung von Teilen des menschlichen oder tierischen Körpers im Rahmen von Diagnose- und Behandlungsverfahren geeignet. Hierbei ist die Ultraschallmessvorrichtung 1, wie allgemein bekannt und üblich, von einer Schutzhülle umgeben und wird in der Nähe der abzubildenden Körperstruktur plaziert. Je nach Anwendung kann die Ultraschallmessvorrichtung 1 sowohl ausserhalb als auch innerhalb des Körpers plaziert werden.

Aufgrund der enorm schnellen, zumindest näherungsweise kontinuierlichen dreidimensionalen Bildgebung sind die Gegenstände der Erfindung insbesondere für solche Anwendungen geeignet, bei denen Instrumente im Körper lokalisiert oder navigiert werden müssen. Hierbei kann es vorteilhaft sein, die Instrumente mit einem Ultraschallmarker zu versehen, der von dem Ultraschallsystem 10 gesteuert wird.

Besonders geeignet ist die erfindungsgemässe Vorrichtung 1 bzw. das erfindungsgemässe System 10 für Anwendungen in der Herzdiagnostik und -chirurgie. Dabei erfolgt die Herzabbildung vorzugsweise transösophagal, das heisst die Ultraschallmessvorrichtung 1 wird in den Ösophagus eingeführt und dort zwecks Bildgebung wie vorne beschrieben betrieben. Sie ermöglicht so eine kontinuierliche dreidimensionale Sicht in das schlagende Herz und damit eine genauere und schnellere Lokalisierung und Manipulation von Instrumenten wie beispielsweise Herzkathetern bei Ablationsbehandlungen, oder die Elektroden-Positionierung für Herzschrittmacher.

Besonders in der Kardiologie ist die erfindungsgemässe Ultraschallmessvorrichtung 1 bzw. das erfindungsgemässe Ultraschallsystem aufgrund der Fähigkeit, dreidimensionale Bilder gleichzeitig von Instrumenten und Körperstrukturen zumindest näherungsweise in Echtzeit zu erstellen, für zahlreiche weitere Anwendungen geeignet, beispielsweise: echographische Bestimmung des Schlagvolumens, dreidimensionale Bewegungsanalyse des Myokards (z. B. nach einem Infarkt), Implantation und Überprüfung der Funktion von (biologischen, unter Röntgenstrahlung nicht sichtbarer) Herzklappenprothesen, Navigation bei minimalinvasiver Herzchirurgie,

## Patentansprüche

1. Ultraschallmessvorrichtung für ein bildgebendes Ultraschallsystem mit einem Trägerkörper (2), der um eine Drehachse (A) dreh- oder schwenkbar ist, und mit einer Mehrzahl von auf dem Trägerkörper (2) angeordneten Wandlerelementen (3) zum Aussenden und Empfangen von Ultraschallsignalen, dadurch gekennzeichnet, dass mindestens zwei der Wandlerelemente (3) bezüglich der Umfangsrichtung des Trägerkörpers (2) und bezüglich der durch die Drehachse (A) festgelegten axialen Richtung relativ zueinander versetzt angeordnet sind.

2. Vorrichtung nach Anspruch 1, wobei die Wandlerelemente (3) in mindestens zwei bezüglich der Umfangsrichtung des Trägerkörpers (2) versetzten Gruppen (30,30a,30b) angeordnet sind, und wobei die Wandlerelemente (3) innerhalb jeder Gruppe (30,30a,30b) bezüglich der axialen Richtung benachbart angeordnet sind.

3. Vorrichtung nach Anspruch 2, wobei die Wandlerelemente (3) innerhalb jeder Gruppe (30,30a,30b) linear angeordnet sind.

4. Vorrichtung nach einem der Ansprüche 2 oder 3, wobei die Wandlerelemente (3) derart angeordnet sind, dass die Zentren (Z) der Wandlerelemente (3) der einen Gruppe (30) bezüglich der Zentren (Z) der Wandlerelemente (3) der oder einer anderen Gruppe (30a,30b) in axialer Richtung versetzt sind.

5. Vorrichtung nach einem der vorangehenden Ansprüche, wobei der Trägerkörper (2) mehrere Messflächen (31,31a,31b) aufweist, die jeweils eine der Gruppen (30,30a,30b) von Wandlerelementen (3) enthalten.

6. Vorrichtung nach Anspruch 5, wobei jeweils benachbarte Messflächen (31,31a,31b) gewinkelt zueinander angeordnet sind.

7. Vorrichtung nach einem der Ansprüche 5 oder 6, wobei die Wandlerelemente (3) derart angeordnet sind, dass die Zentren (Z) der Wandlerelemente (3) der einen Gruppe (30) bezüglich der Zentren (Z) der Wandlerelemente (3) der oder einer anderen Gruppe (30a,30b) in axialer Richtung um einen Betrag (D1,D2) versetzt sind, der im wesentlichen gleich dem Quotienten aus dem axialen Abstand (D) der Zentren (Z) zweier benachbarter Wandlerelemente (3) der gleichen Gruppe und der Anzahl der Messflächen (31,31a,31b) ist.

8. Ultraschallsystem zur Bildgebung, insbesondere zur Erstellung dreidimensionaler Bilder, mit einer Ultraschallmessvorrichtung (1) zum Aussenden und Empfangen von Ultraschallsignalen, mit einer Steuer- und Versorgungseinrichtung (20) für die Ultraschallmessvorrichtung (1) und mit einer Datenverarbeitungseinheit (30), welche die von der Ultraschallmessvorrichtung (1) gelieferten Signale erfasst und derart verarbeitet, dass sie als Bild darstellbar sind, dadurch gekennzeichnet, dass die Ultraschallmessvorrichtung (1) gemäss einem der vorangehenden Ansprüche ausgestaltet ist.

9. System nach Anspruch 8, wobei die Steuer- und Versorgungseinrichtung (20) Mittel umfasst, die so ausgestaltet sind, dass jeweils mehrere Wandlerelemente 3, insbesondere die zu der gleichen Gruppe (30,30a,30b) gehörenden Wandlerelemente (3), parallel oder im wesentlichen parallel betreibbar sind.

10. Verwendung einer Ultraschallmessvorrichtung (1) gemäss einem der Ansprüche 1-7 oder eines Ultraschallsystems (10) gemäss einem der Ansprüche 8 oder 9 zur Darstellung, insbesondere zur dreidimensionalen Darstellung, von Teilen des menschlichen oder tierischen Körpers, insbesondere des Herzens oder Teilen dieses.
